# FASCICULE DE BREVET EUROPEEN

(11) **EP 0 997 135 B1**
(45) Date de publication et mention de la délivrance du brevet: **05.09.2001**
(21) Numéro de dépôt: 99402017.0
(22) Date de dépôt: 09.08.1999
(51) Int. Cl.: A61K 7/043

(54) **Kit de maquillage associant des compositions filmogènes**
Schminkgarnitur mit filmbildenden Zusammensetzungen
Make-up kit with film-forming compositions

(30) Priorité: 30.09.1998 FR 9812234
(43) Date de publication de la demande: 03.05.2000
(73) Titulaire: L'OREAL, 75008 Paris (FR)
(72) Inventeur: Ramin, Roland, 75014 Paris (FR); Brenne, Ingrid, 94240 L'Hay-les-Roses (FR)
(74) Mandataire: Lhoste, Catherine

(56) Documents cités:
- WO-A-92/02204
- GB-A- 2 279 590
- US-A- 5 290 543
- US-A- 5 512 273

## Description

La présente invention se rapporte à un kit de maquillage destiné à un nouveau type de maquillage, associant ceux compositions filmogènes qui peuvent être appliquées sur la peau aussi bien du visage que du corps, sur les lèvres et sur les phanères comme les ongles, les cils, les sourcils ou les cheveux. L'invention a aussi pour objet un procédé de maquillage bicouche.

Chaque composition peut être un vernis-à-ongles, un fard à joue ou à paupières, un fond de teint, un produit de maquillage pour les lèvres ou un produit de maquillage du corps.

Les compositions de vernis à ongles comprennent généralement un polymère filmogène soit solubilisé dans un solvant organique, soit dispersé sous forme de particules dans un milieu aqueux. De tels vernis sort par exemple décrits dans les documents US-A-4158053 et FR-A-2578741.

Ces vernis à ongles, après l'application sur l'ongle d'une ou de plusieurs couches de la composition et après séchage, conduisent généralement à la formation d'un film lisse, continu et homogène, brillant ou mat. Certains films présentent aussi de bonnes propriétés cosmétiques telles qu'une bonne tenue et en particulier une bonne adhérence sur l'ongle, une bonne résistance à l'eau, aux frottements et à l'écaillage.

Avec l'évolution de la mode, les consommateurs, plus exigeants, recherchent de nouveaux produits de maquillage permettant d'obtenir des effets de maquillage originaux ou particuliers. Un besoin subsiste donc de disposer de produit de maquillage dont l'application sur un support comme la peau ou les phanères d'êtres humains permet d'obtenir un effet de maquillage différent des films continus et homogènes actuellement obtenus avec les produits disponibles sur le marché.

La présente invention a donc pour but de proposer une composition de maquillage permettant d'obtenir un maquillage non homogène et/ou un film discontinu sur la peau et sur les phanères, tout en présentant une bonne tenue dans le temps.

La demanderesse a constaté qu'un nouveau type de maquillage de la peau et/ou des phanères pouvait être obtenu à l'aide de l'application de deux compositions filmogènes particulières. Ces compositions permettent d'obtenir un maquillage présentant des petites fissures, ou craquelures, réparties de manière plus ou moins régulière en surface : on obtient ainsi un maquillage présentant un effet craquelé original. En outre, le maquillage présente une bonne adhésion sur le support maquillé et une bonne tenue, notamment aux frottements et à l'écaillage.

De façon plus précise, l'invention a pour objet un kit de maquillage comprenant une première composition comprenant un milieu cosmétiquement acceptable et au moins un premier polymère filmogène, la première composition étant apte à former un premier film ayant une dureté d1 allant de 20 à 85 secondes, et une seconde composition comprenant un milieu cosmétiquement acceptable et au moins un second polymère filmogène, la seconde composition étant apte à former un second film ayant une dureté d2 telle que d2 - d1 soit égal ou supérieur à 10 secondes.

L'invention a encore pour objet un procédé de maquillage de la peau, des lèvres et/ou des phanères d'être humain, consistant à appliquer sur la peau, les lèvres et/ou les phanères une première couche d'une première composition comprenant comprenant un milieu cosmétiquement acceptable et au moins un premier polymère filmogène, la première composition étant apte à former un premier film ayant une dureté d1 allant de 20 à 85 secondes, puis à appliquer sur au moins une partie de la première couche une seconde couche d'une seconde composition filmogène comprenant un milieu cosmétiquement acceptable et au moins un second polymère filmogène, la seconde composition étant apte à former un second film ayant une dureté d2 telle que d2 - d1 soit égal ou supérieur à 10 secondes.

Avantageusement, les deux compositions selon l'invention sont conditionnées dans des compartiments ou récipients distincts, accompagnés de moyens d'application appropriés, identiques ou différents, tels que les pinceaux, les brosses, les plumes, les éponges. Le kit de maquillage contient donc des moyens pour appliquer sur la peau et/ou les phanères la première et la seconde compositions.

Selon l'invention, la seconde couche est appliquée au moins sur une partie de la première couche, voire sur la totalité de la première couche, après séchage de la première couche. Elle peut être appliquée soit à l'une des extrémités de la première couche, soit au milieu, ou encore de façon discontinue notamment sous forme de motifs géométriques, symétriques ou dissymétriques (par exemple sous forme de points, carrés, ronds, étoiles), répartis de façon aléatoire ou ordonnée, à contours précis ou flous.

Cette architecture bicouche peut être adaptée pour tous les produits de maquillage de la peau aussi bien du visage que du scalp et du corps, des muqueuses comme les lèvres et l'intérieur des paupières inférieures, et des phanères comme les ongles. La seconde couche peut être appliquée avec tout type d'instrument (éponge, doigt, pinceau, brosse, plume etc.). Cette architecture peut aussi être appliquée sur les accessoires de maquillage comme les faux ongles, faux cils, perruques, postiches ou encore sur des pastilles ou des patchs adhérents sur la peau ou les lèvres (du type mouches).

L'invention se rapporte aussi à un support maquillé dont le maquillage est susceptible d'être obtenu par le procédé selon l'invention.
L'invention a encore pour objet l'utilisation des première et/ou deuxième compositions telles que définies précédemment pour l'obtention d'un maquillage craquelé en surface, de bonne tenue.
L'invention a également pour objet l'utilisation dans un produit de maquillage d'une composition filmogène comprenant un milieu cosmétiquement acceptable et au moins un premier polymère filmogène, la composition étant apte à former un premier film ayant une dureté d1 allant de 20 à 85 secondes, pour l'obtention d'un maquillage craquelé en surface, de bonne tenue.
L'invention a aussi pour objet l'utilisation dans un produit de maquillage d'une composition filmogène comprenant un milieu cosmétiquement acceptable et au moins un polymère filmogène, destinée à être appliquée sur un premier film ayant une dureté d1 allant de 20 à 85 secondes,
la composition étant apte à former un second film ayant une dureté d2, telle que d2 - d1 soit égal ou supérieur à 10 secondes,
pour l'obtention d'un maquillage craquelé en surface, de bonne tenue.
Les inventeurs ont découverts que l'application de deux compositions de dureté de film distincte selon l'invention permet d'obtenir un maquillage craquelé en surface présentant une bonne adhésion sur le support maquillé et une bonne tenue notamment aux frottements et à l'écaillage. Par ailleurs, l'application de la deuxième composition directement sur le support démaquillé conduit à la formation d'un film craquelé mais le film est très friable et a donc une très mauvaise tenue aux frottements. Ainsi, l'application de la première composition selon l'invention permet d'obtenir un maquillage craquelé présentant une bonne tenue aux frottements.
La dureté du film est mesurée pour une couche de 300 µm d'épaisseur (avant séchage), déposée sur une plaque de verre chauffée à 30 °C et après séchage, pendant 23 heures à atmosphère ambiante puis pendant 1 heure, à 70 % d'humidité relative pour une composition à milieu solvant ou à 50 % d'humidité relative pour une composition à milieu aqueux. La dureté du film obtenu est mesurée selon la norme ASTM D-43-66, ou la norme NF-T 30-016 (décembre 1991), à l'aide d'un pendule de Persoz.
Pour la mise en oeuvre du procédé selon l'invention, la dureté d2 peut être telle que d2 - d1 va de 10 à 360 secondes, et de préférence de 10 à 120 secondes.
De préférence, d1 peut aller de 20 à 85 secondes et d2 peut aller de 30 à 180 secondes. Mieux, d1 peut aller de 20 à 40 secondes et d2 peut aller de 80 à 120 secondes.
Selon le procédé de l'invention, les première et deuxième compositions peuvent comprendre, indépendamment l'une de l'autre, un milieu aqueux ou un milieu solvant organique.
Ainsi, selon une première variante de réalisation de l'invention, les première et deuxième compositions peuvent comprendre un milieu aqueux, identique ou différent.
Selon une deuxième variante de réalisation de l'invention, la première composition peut comprendre un milieu aqueux et la deuxième composition peut comprendre un milieu solvant organique.

Selon une troisième variante de réalisation de l'invention, la première composition peut comprendre un milieu solvant organique et la deuxième composition peut comprendre un milieu aqueux.

Selon une quatrième variante de réalisation de l'invention, les première et deuxième compositions peuvent comprendre un milieu solvant organique, identique ou différent.

Pour favoriser la formation des craquelures ou fissures, notamment réparties de façon régulière à la surface du second film, il est possible d'employer des moyens pour accélérer le séchage de la deuxième couche après l'application de la deuxième composition filmogène. Ces moyens peuvent être par exemple une source de chaleur (sèche cheveux, lampe à halogène) ou d'air pulsé (ventilateur).

Parmi les polymères filmogènes utilisables dans la composition de la présente invention, on peut citer les polymères synthétiques, de type radicalaire ou de type polycondensat, les polymères d'origine naturelle, et leurs mélanges.

Par polymère filmogène radicalaire, on entend un polymère obtenu par polymérisation de monomères à insaturation notamment éthylénique, chaque monomère étant susceptible de s'homopolymériser (à l'inverse des polycondensats). Les polymères filmogènes de type radicalaires peuvent être notamment des polymères, ou des copolymères, vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides.

On utilise de préférence des polymères filmogènes radicalaires anioniques, c'est-à-dire des polymères ayant au moins un monomère à groupement acide.

Comme monomère porteur de groupement acide, on peut utiliser des acides carboxyliques insaturés α,β-éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), notamment des (méth)acrylates d'alkyle, en particulier d'alkyle en C₁-C₂₀, de préférence en C₁-C₈, des (méth)acrylates d'aryle, en particulier d'aryle en C₆-C₁₀, des (méth)acrylates d'hydroxyalkyle, en particulier d'hydroxyalkyle en C₂-C₆.
Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle.
Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.
Parmi les (méth)acrylates d'aryle, on peut citer l'acrylate de benzyle et l'acrylate de phényle.
Les esters de l'acide (méth)acrylique particulièrement préférés sont les (méth)acrylates d'alkyle.

Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Comme amides des monomères acides, on peut par exemple citer les (méth)acrylamides, et notamment les N-alkyl (méth)acrylamides, en particulier d'alkyl en C₂-C₁₂. Parmi les N-alkyl (méth)acrylamides, on peut citer le N-éthyl acrylamide, le N-t-butyl acrylamide et le N-t-octyl acrylamide.

Les polymères filmogènes vinyliques peuvent également résulter de l'homopolymérisation ou de la copolymérisation d'au moins un monomère choisi parmi les esters vinyliques et les monomères styrèniques. En particulier, ces monomères peuvent être polymérisés avec des monomères acides et/ou leurs esters et/ou leurs amides, tels que ceux mentionnés précédemment.
Comme exemple d'esters vinyliques, on peut citer l'acétate de vinyle, le néodécanoate de vinyle, le pivalate de vinyle, le benzoate de vinyle et le t-butyl benzoate de vinyle.
Comme monomères styrèniques, on peut citer le styrène et l'alpha-méthyl styrène.

Il est possible d'utiliser tout monomère connu de l'homme du métier entrant dans les catégories de monomères acryliques et vinyliques (y compris les monomères modifiés par une chaîne siliconée).

Comme polymère filmogène acrylique en dispersion aqueuse utilisable selon l'invention, on peut citer ceux vendus sous les dénominations NEOCRYL XK-90® , NEOCRYL A-1070® , NEOCRYL A-1090® , NEOCRYL BT-62® , NEOCRYL A-1079® , NEOCRYL A-523® par la société ZENECA, DOW LATEX 432® par la société DOW CHEMICAL.

On peut ainsi citer, parmi les polycondensats utilisables comme polymère filmogène, les polyuréthannes anioniques, cationiques, non-ioniques ou amphotères, les polyuréthannes-acryliques, les poly-uréthannes-polyvinylpirrolidones, les polyester-polyuréthannes, les polyéther-polyuréthannes, les polyurées, les polyurée/polyuréthannes, et leurs mélanges.
Le polyuréthanne filmogène peut être, par exemple, un copolymère polyuréthanne, polyurée/uréthanne, ou polyurée, aliphatique, cycloaliphatique ou aromatique, comportant seule ou en mélange :
- au moins une séquence d'origine polyester aliphatique et/ou cycloaliphatique et/ou aromatique, et/ou,
- au moins une séquence siliconée, ramifiée ou non, par exemple polydiméthylsiloxane ou polyméthylphénylsiloxane, et/ou
- au moins une séquence comportant des groupes fluorés.

Comme polymère filmogène polyuréthane en dispersion aqueuse utilisable selon l'invention, on peut notamment citer les polyester-polyuréthanes vendus sous les dénominations "AVALURE UR-405®", "AVALURE UR-410®", "AVALURE UR-425®", "SANCURE 2060®" par la société GOODRICH et les polyéther-polyuréthanes vendus sous les dénominations "SANCURE 878®" par la société GOODRICH, "NEOREZ R 970®" par la société ICI.

Parmi les polycondensats filmogènes, on peut également citer les polyesters, les polyesters amides, les polyesters à chaîne grasse, les polyamides, et les résines époxyesters, les résines résultant de la condensation de formaldéhyde avec une arylsulfonamide, les résines aryl-sulfonamide époxy.

Les polyesters peuvent être obtenus, de façon connue, par polycondensation d'acides dicarboxyliques avec des polyols, notamment des diols.
L'acide dicarboxylique peut être aliphatique, alicyclique ou aromatique. On peut citer comme exemple de tels acides : l'acide oxalique, l'acide malonique, l'acide diméthylmalonique, l'acide succinique, l'acide glutarique, l'acide adipique, l'acide pimélique, l'acide 2,2-diméthylglutarique, l'acide azélaïque, l'acide subérique, l'acide sébacique, l'acide fumarique, l'acide maléique, l'acide itaconique, l'acide phtalique, l'acide dodécanedioïque, l'acide 1,3-cyclohexanedicarboxlique, l'acide 1,4-cyclohexa-nedicarboxylique, l'acide isophtalique, l'acide téréphtalique, l'acide 2,5-norborane dicarboxylique, l'acide diglycolique, l'acide thiodipropionique, l'acide 2,5-naphtalènedicarboxylique, l'acide 2,6-naphta-lènedicarboxylique. Ces monomères acide dicarboxylique peuvent être utilisés seuls ou en combinaison d'au moins deux monomères acide dicarboxylique. Parmi ces monomères, on choisit préférentiellement l'acide phtalique, l'acide isophtalique, l'acide téréphtalique.

Le diol peut être choisi parmi les diols aliphatiques, alicycliques, aromatiques. On utilise de préférence un diol choisi parmi : l'éthylène glycol, le diéthylène glycol, le triéthylène glycol, le 1,3-propanediol, le cyclohexane diméthanol, le 4-butanediol. Comme autres polyols, on peut utiliser le glycérol, le pentaérythritol, le sorbitol, le triméthylol propane.

Les polyesters amides peuvent être obtenus de manière analogue aux polyesters, par. polycondensation de diacides avec des diamines ou des amino alcools. Comme diamine, on peut utiliser l'éthylènediamine, l'hexaméthylènediamine, la méta- ou para-phénylènediamine. Comme aminoalcool, on peut utiliser la monoéthanolaminè.

Le polyester peut en outre comprendre au moins un monomère portant au moins un groupement -SO₃M, avec M représentant un atome d'hydrogène, un ion ammonium NH₄⁺ ou un ion métallique, comme par exemple un ion Na⁺, Li⁺, K+, Mg²⁺, Ca²⁺, Cu²⁺, Fe²⁺, Fe³⁺. On peut utiliser notamment un monomère aromatique bifonctionnel comportant un tel groupement -SO₃M.

Le noyau aromatique du monomère aromatique bifonctionnel portant en outre un groupement -SO₃M tel que décrit ci-dessus peut être choisi par exemple parmi les noyaux benzène, naphtalène, anthracène, diphényl, oxydiphényl, sulfonyldiphényl, méthylènediphényl. On peut citer comme exemple de monomère aromatique bifonctionnel portant en outre un groupement -SO₃M : l'acide sulfoisophtalique, l'acide sulfotéréphtalique, l'acide sulfophtalique, l'acide 4-sulfonaphtalène-2,7-dicarboxylique.
On préfère utiliser dans les compositions objet de l'invention des copolymères à base d'isophtalate/sulfoisophtalate, et plus particulièrement des copolymères obtenus par condensation de di-éthylèneglycol, cyclohexane di-méthanol, acide isophtalique, acide sulfoisophtalique. De tels polymères sont vendus par exemple sous le nom de marque Eastman AQ par la société Eastman Chemical Products.

Les polymères d'origine naturelle, éventuellement modifiés, peuvent être choisis parmi la résine shellac, la gomme de sandaraque, les dammars, les élémis, les copals, les polymères cellulosiques tels que la nitrocellulose, l'acétate de cellulose, l'acétobutyrate de cellulose, l'acétopropionate de cellulose, l'éthyl cellulose, et leurs mélanges.

Avantageusement, la première composition filmogène peut comprendre un copolymère styrène/acrylate dispersé dans un milieu aqueux ou bien encore de la nitrocellulose solubilisée dans un milieu solvant organique, ce milieu solvant organique pouvant comprendre un solvant organique choisi parmi l'acétate d'éthyle, l'acétate de butyle et l'alcool isopropylique ; la seconde composition filmogène peut comprendre un copolymère styrène/acrylate dispersé dans un milieu aqueux.

Les premier et/ou second polymères filmogènes peuvent être solubilisés ou dispersés sous forme de particules dans le milieu cosmétiquement acceptable correspondant de chaque composition selon l'invention. Respectivement, le premier et le deuxième polymère filmogène peuvent être généralement présents en une teneur allant de 1 % à 70 % en poids, par rapport au poids total respectivement de la première et seconde composition, et mieux allant de 10 % à 40 % en poids.

Comme solvant organique utilisable dans l'invention, on peut citer :
- les cétones liquides à température ambiante tels que méthyléthylcétone, méthylisobutylcétone; diisobutylcétone, l'isophorone, la cyclohexanone, l'acétone ;
- les alcools liquides à température ambiante tels que l'éthanol, l'isopropanol, le diacétone alcool, le 2-butoxyéthanol, le cyclohexanol ;
- les glycols liquides à température ambiante tels que l'éthylène glycol, le propylène glycol, le pentylène glycol, le glycérol ;
- les éthers de propylène glycol liquides à température ambiante tels que le monométhyléther de propylène glycol, l'acétate de monométhyl éther de propylène glycol, le mono n-butyl éther de dipropylène glycol ;
- les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle ;
- les éthers liquides à température ambiante tels que le diéthyléther, le diméthyléther ou le dichlorodiéthyléther ;
- les alcanes liquides à température ambiante tels que le décane, l'heptane, le dodécane, le cyclohexane ;
- les composés cycliques aromatiques liquides à température ambiante tels que le toluène et le xylène ;
- les aldéhydes liquides à température ambiante tels que le benzaldéhyde, l'acétaldéhyde
- leurs mélanges.

Ces solvants conviennent plus particulièrement pour le maquillage et le soin des ongles : la composition constitue alors un vernis à ongles ou un produit de soin des ongles.

Dans chaque composition à milieu solvant organique, le solvant organique peut être présent en une teneur allant de 30 à 99 % en poids, par rapport au poids total de chaque composition, et de préférence de 60 % à 90 % en poids.

Lorsque les compositions pour la mise en oeuvre du procédé selon l'invention contiennent un milieu aqueux, ce dernier peut être constitué essentiellement d'eau. La teneur en eau dans chaque composition à milieu aqueux peut aller de 30 à 99 % en poids, par rapport au poids total de chaque composition, et de préférence de 60 % à 90 % en poids.

Lorsque le polymère filmogène ne permet pas d'obtenir seul un film ayant les caractéristiques mentionnées précédemment, il est possible d'ajouter un composé dont la fonction est de modifier les propriétés du polymère filmogène pour obtenir le film ayant la dureté souhaitée. Aussi, selon un mode de réalisation du procédé selon l'invention, on peut ajouter au polymère filmogène au moins un agent auxiliaire de filmification permettant d'obtenir un film ayant les caractéristiques telles que décrites précédemment. L'agent auxiliaire de filmification permet notamment d'obtenir un film ayant la dureté souhaitée pour la mise en oeuvre du procédé seion l'invention.

Un tel agent auxiliaire de filmification peut être choisi parmi tous les composés connus de l'homme du métier comme étant susceptibles de remplir la fonction recherchée, et être notamment choisi parmi les agents plastifiants.

En outre, lorsque l'une des compositions pour la mise en oeuvre du procédé selon l'invention comprend un polymère filmogène sous forme de particules dispersées dans le milieu de la composition, l'agent auxiliaire de filmification peut aussi être choisi parmi les agents de coalescence.

En particulier, on peut citer, seuls ou en mélange, les plastifiants ou agents de coalescence usuels, tels que:
- les glycols et leurs dérivés tels que le diéthylène glycol éthyléther, le diéthylène glycol méthyléther, le diéthylène glycol butyléther ou encore le diéthylène glycol hexyléther, l'éthylène glycol éthyléther, l'éthylène glycol butyléther, l'éthylène glycol hexyléther;
- les esters de glycérol,
- les dérivés de propylène glycol et en particulier le propylène glycol phényléther, le propylène glycol diacétate, le dipropylène glycol butyléther, le tripropylène glycol butyléther, le propylène glycol méthyléther, le dipropylène glycol éthyléther, le tripropylène glycol méthyléther et le diéthylène glycol méthyléther, le propylène glycol butyléther,
- des esters d'acides notamment carboxyliques, tels que des citrates, des phtalates, des adipates, des carbonates, des tartrates, des phosphates, des sébaçates,
- des dérivés oxyéthylénés tels que les huiles oxyéthylénées, notamment les huiles végétales telles que l'huile de ricin; les huiles de silicone,
- des polymères hydrosolubles ayant une température de transition vitreuse faible, inférieure à 25°C, de préférence inférieure à 15°C
- leurs mélanges.

La quantité d'agent auxiliaire de filmification peut être choisie par l'homme du métier sur base de ses connaissances générales, de manière à obtenir un système polymérique conduisant à un film ayant les propriétés mécaniques souhaitées, tout en conservant à la composition des propriétés cosmétiquement acceptables. Pour chaque composition, la teneur en agent auxiliaire de filmification peut par exemple aller de 0,5 % à 20 % en poids, par rapport au poids total de chaque composition, et de préférence de 2 à 10 % en poids.

Chaque composition selon l'invention peut en outre comprendre tout additif connu de l'homme du métier comme étant susceptible d'être incorporé dans une telle composition, tels que les agents épaississants, les agents d'étalement, les agents mouillants, les agents dispersants, les anti-mousses, les conservateurs, les filtres UV, les colorants, les pigments, les actifs, les tensioactifs, les agents hydratants, les parfums, les neutralisants, les stabilisants, les antioxydants. Bien entendu, l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

Chaque composition selon l'invention peut être préparée par l'homme du métier sur la base de ses connaissances générales et selon l'état de la technique.

L'invention est illustrée plus en détail dans les exemples suivants.

### Exemple 1 :

a) On prépare un vernis à ongles ayant la composition 1 suivante pour former la première couche 1 :
   - dispersion aqueuse de copolymère styrènelacrylate 18,8 g MA
   - agent de coalescence 2,3 g
   - agent plastifiant 1,5 g
   - alcool éthylique 5 g
   - agents épaississants 0,4 g
   - conservateurs qs
   - pigments 1,2 g
   - glycérine 0,75 g
   - eau qsp 100 g

   On obtient après séchage un film ayant une dureté mesurée à l'aide du pendule de Persoz comprise entre 20 et 25 secondes.
   Cette composition est appliquée à l'aide d'un pinceau sur la surface des ongles démaquillés, sous forme de monocouche.
b) On prépare un vernis à ongles ayant la composition 2 suivante pour former la deuxième couche 2 :
   - dispersion aqueuse de copolymère styrène/acrylate 19,7 g MA
   - agent de coalescence 2,7 g
   - alcool éthylique 5 g
   - agents épaississants 0,5 g
   - conservateurs qs
   - pigments 3,3 g
   - glycérine 1,8 g
   - eau qsp 100 g

On obtient après séchage un film ayant une dureté mesurée à l'aide du pendule de Persoz comprise entre 60 et 65 secondes.

Cette composition 2 est appliquée à l'aide d'un pinceau sur la totalité de la couche 1 sèche. Après séchage, la couche 2 est craquelée sur toute sa surface de façon régulière et le maquillage craquelé présente une bonne tenue, notamment au frottement avec les doigts. On obtient ainsi un maquillage des ongles de bonne tenue présentant un effet craquelé, fissuré original.

Les craquelures peuvent être également obtenues en accélérant le séchage de la couche 2 au moyen d'une source de chaleur comme un sèche cheveux ou une source d'air pulsé comme un ventilateur.

### Exemple 2 : comparatif

On a appliqué directement sur des ongles démaquillés la composition 2 de l'exemple 1. Après séchage, le film craquèle sur l'ongle mais les craquelures ne résistent pas aux frottements du doigt. Le film craquelé ne tient pas sur l'ongle et se désagrège.

### Exemple 3 :

a) On applique la composition 1 de l'exemple 1 à l'aide d'un pinceau sur la surface des ongles démaquillés, sous forme de monocouche.
b) On prépare un vernis à ongles ayant la composition 2 suivante pour former la deuxième couche 2 :
   - nitrocellulose 11 g
   - plastifiants 5,7 g
   - agents épaississants 1,3 g
   - conservateurs qs
   - pigments 1,2 g
   - solvants (acétate d'éthyle, acétatede butyle, alcool isopropylique) qsp 100 g

On obtient après séchage un film ayant une dureté mesurée à l'aide du pendule de Persoz d'environ 70 secondes.

Cette composition 2 est appliquée à l'aide d'un pinceau sur la totalité de la couche 1 sèche. Après séchage, la couche 2 est craquelée sur toute sa surface de façon régulière et le maquillage craquelé présente une bonne tenue, notamment au frottement avec les doigts.

## Revendications

1. Procédé de maquillage de la peau, des lèvres et/ou des phanères consistant à appliquer sur la peau, les lèvres et/ou les phanères une première couche d'une première composition filmogène comprenant un milieu cosmétiquement acceptable et au moins un premier polymère filmogène, la première composition étant apte à former un premier film ayant une dureté d1 allant de 20 à 85 secondes, puis à appliquer sur au moins une partie de la première couche une seconde couche d'une seconde composition filmogène comprenant un milieu cosmétiquement acceptable et au moins un second polymère filmogène, la seconde composition étant apte à former un second film ayant une dureté d2 telle que d2 - d1 soit égal ou supérieur à 10 secondes, la dureté des films étant mesurée à l'aide d'un pendule de Persoz selon la norme NF-T 30-016.

2. Procédé selon la revendication 1, caractérisé par le fait que d2 - d1 va de 10 à 360 secondes, et de préférence de 10 à 120 secondes.

3. Procédé selon l'une quelconque des revendications précédentes, caractérisé par le fait que la dureté d1 va de 20 à 40 secondes.

4. Procédé selon l'une quelconque des revendications précédentes, caractérisé par le fait que la dureté d2 va de 30 à 180 secondes.

5. Procédé selon l'une quelconque des revendications précédentes, caractérisé par le fait que la dureté d2 va de 80 à 120 secondes.

6. Procédé selon l'une quelconque des revendications précédentes, caractérisé par le fait que la première et/ou la deuxième composition comprennent un milieu solvant organique.

7. Procédé selon l'une quelconque des revendications précédentes, caractérisé par le fait que la première et/ou deuxième composition comprennent un milieu aqueux.

8. Procédé selon l'une quelconque des revendications précédentes, caractérisé par le fait que le premier et/ou second polymère filmogène sont solubilisés dans le milieu cosmétiquement acceptable correspondant.

9. Procédé selon l'une quelconque des revendications précédentes, caractérisé par le fait que le premier et/ou deuxième polymère filmogène sont dispersés sous forme de particules dans le milieu cosmétiquement acceptable correspondant.

10. Procédé selon l'une quelconque des revendications précédentes, caractérisé par le fait que les premier et deuxième polymères filmogènes sont choisis, indifféremment l'un de l'autre, dans le groupe formé par les polymères radicalaires, les polycondensats et les polymères d'origine naturelle.

11. Procédé selon l'une quelconque des revendications précédentes, caractérisé par le fait que les premier et deuxième polymères filmogènes sont choisis, indifféremment l'un de l'autre, dans le groupe formé pal les polymères vinyliques, les polyuréthanes, les polyesters, les polymères cellulosiques.

12. Procédé selon l'une quelconque des revendications précédentes, caractérisé par le fait que la teneur en respectivement premier et second polymère filmogène va de 1 % à 70 % en poids, par rapport au poids total respectivement de la première et seconde composition, et de préférence de 10 % à 40 % en poids.

13. Procédé selon l'une quelconque des revendications précédentes, caractérisé par le fait que la première et/ou la deuxième composition comprennent au moins un agent auxiliaire de filmification.

14. Procédé selon la revendication 13, caractérisé par le fait que l'agent auxiliaire de filmification est présent en une teneur allant de 0,5 % à 20 % en poids, par rapport au poids total de chaque première et seconde composition, et de préférence de 2 à 10 % en poids.

15. Procédé selon l'une quelconque des revendications précédentes, caractérisé par le fait que la première et/ou la deuxième composition comprennent au moins un agent plastifiant.

16. Procédé selon l'une quelconque des revendications 9 à 15, caractérisé par le fait que la première et/ou la deuxième composition comprennent au moins un agent de coalescence.

17. Procédé selon l'une quelconque des revendications précédentes, caractérisé par le fait que la première et/ou seconde composition comprennent en outre au moins un additif choisi dans le groupe formé par les agents épaississants, les agents d'étalement, les agents mouillants, les agents dispersants, les anti-mousses, les conservateurs, les filtres UV, les colorants, les pigments, les actifs, les tensioactifs, les agents hydratants, les parfums, les neutralisants, les stabilisants, les antioxydants.

18. Procédé selon l'une quelconque des revendications précédentes, caractérisé par le fait que la première et/ou seconde composition sont sous la forme de vernis à ongles, de fard à joue ou à paupières, de fond de teint, de produit de maquillage des lèvres ou du corps.

19. Procédé selon l'une quelconque des revendications précédentes, caractérisé par le fait que le séchage de la deuxième couche est accéléré à l'aide d'une source de chaleur ou d'air pulsé.

20. Procédé selon l'une quelconque des revendications précédentes, caractérisé par le fait que la première composition filmogène comprend un copolymère styrène/acrylate dispersé dans un milieu aqueux ou de la nitrocellulose solubilisée dans un milieu solvant organique.

21. Procédé selon l'une quelconque des revendications précédentes, caractérisé par le fait que la seconde composition filmogène comprend un copolymère styrène/acrylate dispersé dans un milieu aqueux.

22. Produit de maquillage susceptible d'être obtenu selon le procédé tel que défini selon l'une des revendications 1 à 21.

23. Kit de maquillage comprenant :
- une première composition comprenant au moins un premier polymère filmogène dans un milieu cosmétiquement acceptable, la première composition étant apte à former un premier film ayant une dureté d1 allant de 20 à 85 secondes, et
- une deuxième composition comprenant au moins un second polymère filmogène dans un milieu cosmétiquement acceptable, la deuxième composition étant apte à former un second film ayant une dureté d2 telle que d2 - d1 soit égal ou supérieur à 10 secondes,
la dureté des films étant mesurée à l'aide d'un pendule de Persoz selon la norme NF-T-30-016.

24. Kit de maquillage selon la revendication 23, caractérisé par le fait que d2 - d1 va de 10 à 360 secondes, et de préférence de 10 à 120 secondes.

25. Kit de maquillage selon l'une des revendications 23 ou 24, caractérisé par le fait que la dureté d1 va de 20 à 40 secondes.

26. Kit de maquillage selon l'une quelconque des revendications 23 à 25, caractérisé par le fait que la dureté d2 va de 30 à 180 secondes.

27. Kit de maquillage selon l'une quelconque des revendications 23 à 26, caractérisé par le fait que la dureté d2 va de 80 à 120 secondes.

28. Kit de maquillage selon l'une quelconque des revendications 23 à 27, caractérisé par le fait que la première et/ou la deuxième composition comprennent un milieu solvant organique.

29. Kit de maquillage selon l'une quelconque des revendications 23 à 27, caractérisé par le fait que la première et/ou deuxième composition comprennent un milieu aqueux.

30. Kit de maquillage selon l'une quelconque des revendications 23 à 29, caractérisé par le fait que le premier et/ou second polymère filmogène sont solubilisés dans le milieu cosmétiquement acceptable correspondant.

31. Kit de maquillage selon l'une quelconque des revendications 23 à 30, caractérisé par le fait que le premier et/ou deuxième polymère filmogène sont dispersés sous forme de particules dans le milieu cosmétiquement acceptable correspondant.

32. Kit de maquillage selon l'une quelconque des revendications 23 à 31, caractérisé par le fait que les premier et deuxième polymères filmogènes sont choisis, indifféremment l'un de l'autre, dans le groupe formé par les polymères radicalaires, les polycondensats et les polymères d'origine naturelle.

33. Kit de maquillage selon l'une quelconque des revendications 23 à 32, caractérisé par le fait que les premier et deuxième polymères filmogènes sont choisis, indifféremment l'un de l'autre, dans le groupe formé par les polymères vinyliques, les polyuréthanes, les polyesters, les polymères cellulosiques.

34. Kit de maquillage selon l'une quelconque des revendications 23 à 33, caractérisé par le fait que la teneur en respectivement premier et second polymère filmogène va de 1 % à 70 % en poids, par rapport au poids total respectivement de la première et seconde composition, et de préférence de 10 % à 40 % en poids.

35. Kit de maquillage selon l'une quelconque des revendications 23 à 34, caractérisé par le fait que la première et/ou la deuxième composition comprennent au moins un agent auxiliaire de filmification.

36. Kit de maquillage selon la revendication 35, caractérisé par le fait que l'agent auxiliaire de filmification est présent en une teneur allant de 0,5 % à 20 % en poids, par rapport au poids total de chaque première et seconde composition, et de préférence de 2 à 10 % en poids.

37. Kit de maquillage selon l'une quelconque des revendications 23 à 36, caractérisé par le fait que la première et/ou la deuxième composition comprennent au moins un agent plastifiant.

38. Kit de maquillage selon l'une quelconque des revendications 31 à 37, caractérisé par le fait que la première et/ou la deuxième composition comprennent au moins un agent de coalescence.

39. Kit de maquillage selon l'une quelconque des revendications 23 à 38, caractérisé par le fait que la première et/ou seconde composition comprennent en outre au moins un additif choisi dans le groupe formé par les agents épaississants, les agents d'étalement, les agents mouillants, les agents dispersants, les anti-mousses, les conservateurs, les filtres UV, les colorants, les pigments, les actifs, les tensioactifs, les agents hydratants, les parfums, les neutralisants, les stabilisants, les antioxydants.

40. Kit de maquillage selon l'une quelconque des revendications 23 à 39, caractérisé par le fait que la première et/ou seconde composition sont sous la forme de vernis à ongles, de fard à joue ou à paupières, de fond de teint, de produit de maquillage des lèvres ou du corps.

41. Kit de maquillage selon l'une quelconque des revendications 23 à 40, caractérisé par le fait qu'il contient des moyens pour appliquer sur la peau et/ou les phanères la première et la seconde compositions.

42. Kit de maquillage selon la revendication 41, caractérisé par le fait que les moyens sont choisis dans le groupe formé par les pinceaux, les brosses, les plumes, les éponges.

43. Kit de maquillage selon l'une des revendications 23 à 42, caractérisé par le fait que les première et seconde compositions sont conditionnées dans des compartiments ou récipients distincts.

44. Kit de maquillage selon l'une quelconque des revendications 23 à 43, caractérisé par le fait que la première composition filmogène comprend un copolymère styrène/acrylate dispersé dans un milieu aqueux ou de la nitrocellulose solubilisée dans un milieu solvant organique.

45. Kit de maquillage selon l'une quelconque des revendications 23 à 44, caractérisé par le fait que la seconde composition filmogène comprend un copolymère styrène/acrylate dispersé dans un milieu aqueux.

46. Support maquillé comprenant un maquillage susceptible d'être obtenu selon le procédé tel que défini selon l'une des revendications 1 à 21.

47. Support selon la revendication 46, caractérisé par le fait qu'il se présente sous forme de faux ongles, de faux cils, de postiches, de perruques, de pastilles, de patchs adhérents sur la peau ou les lèvres.

48. Utilisation d'une première composition filmogène comprenant un milieu cosmétiquement acceptable et au moins un premier polymère filmogène, la première composition étant apte à former un premier film ayant une dureté d1 allant de 20 à 85 secondes,
et d'une deuxième composition filmogène comprenant un milieu cosmétiquement acceptable et au moins un second polymère filmogène, la seconde composition étant apte à former un second film ayant une dureté d2 telle que d2 - d1 soit égal ou supérieur à 10 secondes,
la dureté des films étant mesurée à l'aide d'un pendule de Persoz selon la norme NF-T-30-016,
pour l'obtention d'un maquillage craquelé en surface, de bonne tenue.

49. Utilisation selon la revendication 48, caractérisée par le fait que la première composition filmogène comprend un copolymère styrène/acrylate dispersé dans un milieu aqueux ou de la nitrocellulose solubilisée dans un milieu solvant organique,
et que la seconde composition filmogène comprend un copolymère styrène/acrylate dispersé dans un milieu aqueux.

50. Utilisation d'une composition filmogène comprenant un milieu cosmétiquement acceptable et au moins un premier polymère filmogène, la composition étant apte à former un premier film ayant une dureté d1 allant de 20 à 85 secondes, mesurée à l'aide d'un pendule de Persoz selon la norme NF-T-30-016,
pour l'obtention d'un maquillage craquelé en surface, de bonne tenue.

51. Utilisation selon la revendication 50, caractérisée par le fait que
la première composition filmogène comprend un copolymère styrène/acrylate dispersé dans un milieu aqueux ou de la nitrocellulose solubilisée dans un milieu solvant organique.

52. Utilisation dans un produit de maquillage d'une composition filmogène comprenant un milieu cosmétiquement acceptable et au moins un polymère filmogène, destinée à être appliquée sur un premier film ayant une dureté d1 allant de 20 à 85 secondes,
la composition étant apte à former un second film ayant une dureté d2, telle que d2 - d1 soit égal ou supérieur à 10 secondes, la dureté des films étant mesurée à l'aide d'un pendule de Persoz selon la norme NF-T-30-016,
pour l'obtention d'un maquillage craquelé en surface, de bonne tenue.

53. Utilisation selon la revendication 52, caractérisé par le fait que la composition filmogène comprend un copolymère styrène/acrylate dispersé dans un milieu aqueux.

## Claims

1. Method of making up the skin, lips and/or exoskeletal appendages, which consists in applying to the skin, lips and/or exoskeletal appendages a first layer of a first film--forming composition comprising a cosmetically acceptable medium and at least one first film-forming polymer, the first composition being capable of forming a first film having a hardness d1 ranging from 20 to 85 seconds, then in applying to at least one part of the first layer a second layer of a second film-forming composition comprising a cosmetically acceptable medium and at least one second film-forming polymer, the second composition being capable of forming a second film having a hardness d2 such that d2 - d1 is greater than or equal to 10 seconds, the hardness of the films being measured with the aid of a Persoz pendulum in accordance with the standard NF-T-30-016.

2. Method according to Claim 1, **characterized in that** d2 - d1 ranges from 10 to 360 seconds and, preferably, from 10 to 120 seconds.

3. Method according to either of the preceding claims, **characterized in that** the hardness d1 ranges from 20 to 40 seconds.

4. Method according to any one of the preceding claims, **characterized in that** the hardness d2 ranges from 30 to 180 seconds.

5. Method according to any one of the preceding claims, **characterized in that** the hardness d2 ranges from 80 to 120 seconds.

6. Method according to any one of the preceding claims, **characterized in that** the first and/or the second composition comprise/comprises an organic solvent medium.

7. Method according to any one of the preceding claims, **characterized in that** the first and/or second composition comprise/comprises an aqueous medium.

8. Method according to any one of the preceding claims, **characterized in that** the first and/or second film-forming polymer are/is dissolved in the corresponding cosmetically acceptable medium.

9. Method according to any one of the preceding claims, **characterized in that** the first and/or second film-forming polymer are/is dispersed in particle form in the corresponding cosmetically acceptable medium.

10. Method according to any one of the preceding claims, **characterized in that** the first and second film-forming polymers are selected independently of one another from the group consisting of free-radical polymers, polycondensates and polymers of natural origin.

11. Method according to any one of the preceding claims, **characterized in that** the first and second film-forming polymers are selected independently of one another from the group consisting of vinyl polymers, polyurethanes, polyesters and cellulosic polymers..

12. Method according to any one of the preceding claims, **characterized in that** the amount of, respectively, first and second film-forming polymer ranges from 1% to 70% by weight relative to the total weight, respectively, of the first and second composition, and preferably from 10% to 40% by weight.

13. Method according to any one of the preceding claims, **characterized in that** the first and/or the second composition comprise/comprises at least one film-forming auxiliary.

14. Method according to Claim 13, **characterized in that** the film-forming auxiliary is present in an amount ranging from 0.5% to 20% by weight relative to the total weight of each first and second composition, and preferably from 2 to 10% by weight.

15. Method according to any one of the preceding claims, **characterized in that** the first and/or the second composition comprise/comprises at least one plasticizer.

16. Method according to any one of Claims 9 to 15, **characterized in that** the first and/or the second composition comprise/comprises at least one coalescent.

17. Method according to any one of the preceding claims, **characterized in that** the first and/or second composition additionally comprise/comprises at least one additive selected from the group consisting of thickeners, levelling agents, wetting agents, dispersants, antifoams, preservatives, UV filters, dyes, pigments, active substances, surfactants, moisturizers, perfumes, neutralizing agents, stabilizers and antioxidants.

18. Method according to any one of the preceding claims, **characterized in that** the first and/or second composition are/is in the form of nail varnish, rouge or eyeshadow, foundation or a lip or body makeup product.

19. Method according to any one of the preceding claims, **characterized in that** the drying of the second layer is accelerated with the aid of a source of heat or of pulsed air.

20. Method according to any one of the preceding claims, **characterized in that** the first film-forming composition comprises a styrene/acrylate copolymer dispersed in an aqueous medium, or nitrocellulose dissolved in an organic solvent medium.

21. Method according to any one of the preceding claims, **characterised in that** the second film-forming composition comprises a styrene/acrylate copolymer dispersed in an aqueous medium.

22. Makeup product obtainable in accordance with the method as defined in one of Claims 1 to 21.

23. Makeup kit comprising
- a first composition comprising at least one first film-forming polymer in a cosmetically acceptable medium, the first composition being capable of forming a first film having a hardness d1 ranging from 20 to 85 seconds, and
- a second composition comprising at least one second film-forming polymer in a cosmetically acceptable medium, the second composition being capable of forming a second film having a hardness d2 such that d2 - d1 is greater than or equal to 10 seconds,
the hardness of the films being measured with the aid of a Persoz pendulum in accordance with the standard MF-T-30-016.

24. Makeup kit according to Claim 23, **characterized in that** d2 - d1 ranges from 10 to 360 seconds and, preferably, from 10 to 120 seconds.

25. Makeup kit according to either of Claims 23 or 24, **characterized in that** the hardness d1 ranges from 20 to 40 seconds.

26. Makeup kit according to any one of Claims 23 to 25, **characterized in that** the hardness d2 ranges from 30 to 180 seconds.

27. Makeup kit according to any one of Claims 23 to 26, **characterized in that** the hardness d2 ranges from 80 to 120 seconds.

28. Makeup kit according to any one of Claims 23 to 27, **characterized in that** the first and/or the second composition comprise/comprises an organic solvent medium.

29. Makeup kit according to any one of Claims 23 to 27, **characterized in that** the first and/or second composition comprise/comprises an aqueous medium.

30. Makeup kit according to any one of Claims 23 to 29, **characterized in that** the first and/or second film-forming polymer are/is dissolved in the corresponding cosmetically acceptable medium.

31. Makeup kit according to any one of Claims 23 to 30, **characterized in that** the first and/or second film-forming polymer are/is dispersed in particle form in the corresponding cosmetically acceptable medium.

32. Makeup kit according to any one of Claims 23 to 31, **characterized in that** the first and second film-forming polymers are selected independently of one another from the group consisting of free-radical polymers, polycondensates and polymers of natural origin.

33. Makeup kit according to any one of Claims 23 to 32, **characterized in that** the first and second film-forming polymers are selected independently of one another from the group consisting of vinyl polymers, polyurethanes, polyesters and cellulosic polymers.

34. Makeup kit according to any one of Claims 23 to 33, **characterized in that** the amount of, respectively, first and second film-forming polymer ranges from 1% to 70% by weight relative to the total weight, respectively, of the first and second composition, and preferably from 10% to 40% by weight.

35. Makeup kit according to any one of Claims 23 to 34, **characterized in that** the first and/or the second composition comprise/comprises at least one film-forming auxiliary.

36. Makeup kit according to Claim 35, **characterized in that** the film-forming auxiliary is present in an amount ranging from 0.5% to 20% by weight relative to the total weight of each first and second composition, and preferably from 2 to 10% by weight.

37. Makeup kit according to any one of Claims 23 to 36, **characterized in that** the first and/or the second composition comprise/comprises at least one plasticizer.

38. Makeup kit according to any one of Claims 31 to 37, **characterized in that** the first and/or the second composition comprise/comprises at least one coalescent.

39. Makeup kit according to any one of Claims 23 to 38, **characterized in that** the first and/or second composition additionally comprise/comprises at least one additive selected from the group consisting of thickeners, levelling agents, wetting agents, dispersants, antifoams, preservatives, UV filters, dyes, pigments, active substances, surfactants, moisturizers, perfumes, neutralizing agents, stabilizers and antioxidants.

40. Makeup kit according to any one of Claims 23 to 39, **characterized in that** the first and/or second composition are/is in the form of nail varnish, rouge or eyeshadow, foundation or a lip or body makeup product.

41. Makeup kit according to any one of Claims 23 to 40, **characterized in that** it comprises means for applying the first and second compositions to the skin and/or the exoskeletal appendages.

42. Makeup kit according to Claim 41, **characterized in that** the means are selected from the group consisting of coarse and fine brushes, pens and sponges.

43. Makeup kit according to one of Claims 23 to 42, **characterized in that** the first and second compositions are packaged in separate containers or compartments.

44. Makeup kit according to any one of Claims 23 to 43, **characterized in that** the first film-forming composition comprises a styrene/acrylate copolymer dispersed in an aqueous medium, or nitrocellulose dissolved in an organic solvent medium.

45. Makeup kit according to any one of Claims 23 to 44, **characterized in that** the second film-forming composition comprises a styrene/acrylate copolymer dispersed in an aqueous medium.

46. Made-up substrate comprising a makeup obtainable in accordance with the method as defined in one of Claims 1 to 21.

47. Substrate according to Claim 46, **characterized in that** it is in the form of false nails, false eyelashes, hairpieces, wigs, inserts or adhesive patches on the skin or lips.

48. Use of a first film-forming composition comprising a cosmetically acceptable medium and at least one first film-forming polymer, the first composition being capable of forming a first film having a hardness d1 ranging from 20 to 85 seconds,
and of a second film-forming composition comprising a cosmetically acceptable medium and at least one second film-forming polymer, the second composition being capable of forming a second film having a hardness d2 such that d2 - d1 is greater than or equal to 10 seconds,
the hardness of the films being measured with the aid of a Persoz pendulum in accordance with the standard NF-T-30-016,
to obtain a crackle-surface makeup having good hold.

49. Use according to Claim 48, **characterized in that** the first film-forming composition comprises a styrene/acrylate copolymer dispersed in an aqueous medium or nitrocellulose dissolved in an organic solvent medium and
in that the second film-forming composition comprises a styrene/acrylate copolymer dispersed in an aqueous medium.

50. Use of a film-forming composition comprising a cosmetically acceptable medium and at least one first film-forming polymer, the composition being capable of forming a first film having a hardness d1 ranging from 20 to 85 seconds, measured with the aid of a Persoz pendulum in accordance with the standard NF-T-30-016, to obtain a crackle-surface makeup having good hold.

51. Use according to Claim 50, **characterized in that** the first film-forming composition comprises a styrene/acrylate copolymer dispersed in an aqueous medium or nitrocellulose dissolved in an organic solvent medium.

52. Use in a makeup product of a film-forming composition comprising a cosmetically acceptable medium and at least one film-forming polymer, intended for application to a first film having a hardness d1 ranging from 20 to 85 seconds, . the composition being capable of forming a second film having a hardness d2 such that d2 - d1 is greater than or equal to 10 seconds, the hardness of the films being measured with the aid of a Persoz pendulum in accordance with the standard NF-T-30-016, to obtain a crackle-surface makeup having good hold.

53. Use according to Claim 52, **characterized in that** the film-forming composition comprises a styrene/acrylate copolymer dispersed in an aqueous medium.

## Patentansprüche

1. Verfahren zum Schminken der Haut, der Lippen und/oder der Hautanhangsgebilde, das darin besteht, auf die Haut, die Lippen und/oder die Hautanhangsgebilde eine erste Schicht einer ersten filmbildenden Zusammensetzung aufzutragen, die ein kosmetisch akzeptables Medium und mindestens ein erstes filmbildendes Polymer enthält, wobei die erste Zusammensetzung zur Bildung eines ersten Films mit einer Härte d1 von 20 bis 85 Sekunden befähigt ist, und anschließend zumindest auf einen Teil der ersten Schicht eine zweite Schicht einer zweiten filmbildenden Zusammensetzung aufzutragen, die ein kosmetisch akzeptables Medium und mindestens ein zweites filmbildendes Polymer enthält, wobei die zweite Zusammensetzung zur Bildung eines zweiten Films mit einer Härte d2 befähigt ist, die so ist, dass d2 - d1 größer oder gleich 10 Sekunden ist, wobei die Härte der Filme gemäß der Norm NF-T 30-016 mit einem Pendelschlagwerk nach Persoz bestimmt wird.

2. Verfahren nach Anspruch 1, **dadurch gekennzeichnet**, dass d2 - d1 im Bereich von 10 bis 360 Sekunden und vorzugsweise von 10 bis 120 Sekunden liegt.

3. Verfahren nach einem der vorhergehenden Ansprüche, **dadurch gekennzeichnet**, dass die Härte d1 im Bereich von 20 bis 40 Sekunden liegt.

4. Verfahren nach einem der vorhergehenden Ansprüche, **dadurch gekennzeichnet**, dass die Härte d2 im Bereich von 30 bis 180 Sekunden liegt.

5. Verfahren nach einem der vorhergehenden Ansprüche, **dadurch gekennzeichnet**, dass die Härte d2 im Bereich von 80 bis 120 Sekunden liegt.

6. Verfahren nach einem der vorhergehenden Ansprüche, **dadurch gekennzeichnet**, dass die erste und/oder die zweite Zusammensetzung ein organisches Lösemittelmedium enthält.

7. Verfahren nach einem der vorhergehenden Ansprüche, **dadurch gekennzeichnet**, dass die erste und/oder die zweite Zusammensetzung ein wässeriges Medium enthält.

8. Verfahren nach einem der vorhergehenden Ansprüche, **dadurch gekennzeichnet**, dass das erste und/oder das zweite filmbildende Polymer in dem entsprechenden kosmetisch akzeptablen Medium solubilisiert ist.

9. Verfahren nach einem der vorhergehenden Ansprüche, **dadurch gekennzeichnet**, dass das erste und/oder das zweite filmbildende Polymer in dem entsprechenden kosmetisch akzeptablen Medium in Form von Partikeln dispergiert ist.

10. Verfahren nach einem der vorhergehenden Ansprüche, **dadurch gekennzeichnet**, dass das erste und das zweite filmbildende Polymer unabhängig voneinander unter durch radikalische Polymerisation hergestellten Polymeren, Polykondensaten und Polymeren natürlichen Ursprungs ausgewählt sind.

11. Verfahren nach einem der vorhergehenden Ansprüche, **dadurch gekennzeichnet**, dass das erste und das zweite filmbildende Polymer unabhängig voneinander unter Vinylpolymeren, Polyurethanen, Polyestem und Cellulosepolymeren ausgewählt sind.

12. Verfahren nach einem der vorhergehenden Ansprüche, **dadurch gekennzeichnet**, dass der Mengenanteil des ersten bzw. des zweiten filmbildenden Polymers im Bereich von 1 bis 70 Gew.-% und vorzugsweise von 10 bis 40 Gew.-%, bezogen auf das Gesamtgewicht der ersten bzw. der zweiten Zusammensetzung, liegt.

13. Verfahren nach einem der vorhergehenden Ansprüche, **dadurch gekennzeichnet**, dass die erste und/oder die zweite Zusammensetzung mindestens ein Hilfsmittel für die Filmbildung enthält.

14. Verfahren nach Anspruch 13, **dadurch gekennzeichnet**, dass das Hilfsmittel für die Filmbildung in einem Mengenanteil im Bereich von 0,5 bis 20 Gew.-% und vorzugsweise von 2 bis 10 Gew.-%, bezogen auf das Gesamtgewicht der jeweiligen ersten und zweiten Zusammensetzung, vorliegt.

15. Verfahren nach einem der vorhergehenden Ansprüche, **dadurch gekennzeichnet**, dass die erste und/oder die zweite Zusammensetzung mindestens einen Weichmacher enthält.

16. Verfahren nach einem der Ansprüche 9 bis 15, **dadurch gekennzeichnet**, dass die erste und/oder die zweite Zusammensetzung mindestens ein Koaleszenzmittel enthält.

17. Verfahren nach einem der vorhergehenden Ansprüche, **dadurch gekennzeichnet**, dass die erste und/oder die zweite Zusammensetzung außerdem mindestens einen Zusatzstoff enthält, der unter Verdickungsmitteln, Spreitmitteln, Netzmitteln, Dispergiermitteln, Schaumverhütungsmitteln, Konservierungsmitteln, UV-Filtern, Färbemitteln, Pigmenten, Wirkstoffen, grenzflächenaktiven Stoffen, Hydratisierungsmitteln, Parfums, Neutralisationsmitteln, Stabilisatoren und Antioxidantien ausgewählt ist.

18. Verfahren nach einem der vorhergehenden Ansprüche, **dadurch gekennzeichnet**, dass die erste und/oder die zweite Zusammensetzung in Form eines Nagellacks, eines Wangenrouges, eines Lidschattens, eines Make-ups oder in Form eines Produktes zum Schminken der Lippen oder des Körpers vorliegt.

19. Verfahren nach einem der vorhergehenden Ansprüche, **dadurch gekennzeichnet**, dass der Trocknungsvorgang der zweiten Schicht mit einer Wärmequelle oder mit einem Gebläse beschleunigt wird.

20. Verfahren nach einem der vorhergehenden Ansprüche, **dadurch gekennzeichnet**, dass die erste filmbildende Zusammensetzung ein in einem wässerigen Medium dispergiertes Styrol/Acrylat-Copolymer oder in einem organischen Lösemittelmedium solubilisierte Nitrocellulose enthält.

21. Verfahren nach einem der vorhergehenden Ansprüche, **dadurch gekennzeichnet**, dass die zweite filmbildende Zusammensetzung ein Styrol/Acrylat-Copolymer enthält, das in einem wässerigen Medium dispergiert ist.

22. Schminkprodukt, das gemäß dem Verfahren nach einem der Ansprüche 1 bis 21 erhalten werden kann.

23. Kit zum Schminken, der aufweist:
- eine erste Zusammensetzung, die in einem kosmetisch akzeptablen Medium mindestens ein erstes filmbildendes Polymer enthält, wobei die erste Zusammensetzung zur Bildung eines ersten Films mit einer Härte d1 im Bereich 20 bis 85 Sekunden befähigt ist, und
- eine zweite Zusammensetzung, die in einem kosmetisch akzeptablen Medium mindestens ein zweites filmbildendes Polymer enthält, wobei die zweite Zusammensetzung zur Bildung eines zweiten Films mit einer Härte d2 befähigt ist, die so ist, dass d2 - d1 größer oder gleich 10 Sekunden ist,
wobei die Härte der Filme gemäß der Norm NF-T 30-016 mit einem Pendelschlagwerk nach Persoz bestimmt wird.

24. Kit zum Schminken nach Anspruch 23, **dadurch gekennzeichnet**, dass d2 - d1 im Bereich von 10 bis 360 Sekunden und vorzugsweise von 10 bis 120 Sekunden liegt.

25. Kit zum Schminken nach einem der Ansprüche 23 oder 24, **dadurch gekennzeichnet,** dass die Härte d1 im Bereich von 20 bis 40 Sekunden liegt.

26. Kit zum Schminken nach einem der Ansprüche 23 bis 25, **dadurch gekennzeichnet**, dass die Härte d2 im Bereich von 30 bis 180 Sekunden liegt.

27. Kit zum Schminken nach einem der Ansprüche 23 bis 26, **dadurch gekennzeichnet**, dass die Härte d2 im Bereich von 80 bis 120 Sekunden liegt.

28. Kit zum Schminken nach einem der Ansprüche 23 bis 27, **dadurch gekennzeichnet**, dass die erste und/oder die zweite Zusammensetzung ein organisches Lösemittelmedium enthält.

29. Kit zum Schminken nach einem der Ansprüche 23 bis 27, **dadurch gekennzeichnet**, dass die erste und/oder die zweite Zusammensetzung ein wässeriges Medium enthält.

30. Kit zum Schminken nach einem der Ansprüche 23 bis 29, **dadurch gekennzeichnet**, dass das erste und/oder das zweite filmbildende Polymer in dem entsprechenden kosmetisch akzeptablen Medium solubilisiert ist.

31. Kit zum Schminken nach einem der Ansprüche 23 bis 30, **dadurch gekennzeichnet**, dass das erste und/oder das zweite filmbildende Polymer in dem entsprechenden kosmetisch akzeptablen Medium in Form von Partikeln dispergiert ist.

32. Kit zum Schminken nach einem der Ansprüche 23 bis 31, **dadurch gekennzeichnet**, dass das erste und das zweite filmbildende Polymer unabhängig voneinander unter durch radikalische Polymerisation hergestellten Polymeren, Polykondensaten und Polymeren natürlischen Ursprungs ausgewählt sind.

33. Kit zum Schminken nach einem der Ansprüche 23 bis 32, **dadurch gekennzeichnet**, dass das erste und das zweite filmbildende Polymer unabhängig voneinander unter Vinylpolymeren, Polyurethanen, Polyestern und Cellulosepolymeren ausgewählt sind.

34. Kit zum Schminken nach einem der Ansprüche 23 bis 33, **dadurch gekennzeichnet**, dass der Mengenanteil des ersten bzw. des zweiten filmbildenden Polymers im Bereich von 1 bis 70 Gew.-% und vorzugsweise von 10 bis 40 Gew.-%, bezogen auf das Gesamtgewicht der ersten bzw. der zweiten Zusammensetzung, liegt.

35. Kit zum Schminken nach einem der Ansprüche 23 bis 34, **dadurch gekennzeichnet**, dass die erste und/oder die zweite Zusammensetzung mindestens ein Hilfsmittel für die Filmbildung enthält.

36. Kit zum Schminken nach Anspruch 35, **dadurch gekennzeichnet**, dass das Hilfsmittel für die Filmbildung in einem Mengenanteil im Bereich von 0,5 bis 20 Gew.-% und vorzugsweise von 2 bis 10 Gew.-%, bezogen auf das Gesamtgewicht der jeweiligen ersten und zweiten Zusammensetzung, vorliegt.

37. Kit zum Schminken nach einem der Ansprüche 23 bis 36, **dadurch gekennzeichnet**, dass die erste und/oder die zweite Zusammensetzung mindestens einen Weichmacher enthält.

38. Kit zum Schminken nach einem der Ansprüche 31 bis 37, **dadurch gekennzeichnet**, dass die erste und/oder die zweite Zusammensetzung mindestens ein Koaleszenzmittel enthält.

39. Kit zum Schminken nach einem der Ansprüche 23 bis 38, **dadurch gekennzeichnet**, dass die erste und/oder die zweite Zusammensetzung außerdem mindestens einen Zusatzstoff enthält, der unter Verdickungsmitteln, Spreitmitteln, Netzmitteln, Dispergiermitteln, Schaumverhütungsmitteln, Konservierungsmitteln, UV-Filtern, Färbemitteln, Pigmenten, Wirkstoffen, grenzflächenaktiven Stoffen, Hydratisierungsmitteln, Parfums, Neutralisationsmitteln, Stabilisatoren und Antioxidantien ausgewählt ist.

40. Kit zum Schminken nach einem der Ansprüche 23 bis 39, **dadurch gekennzeichnet**, dass die erste und/oder die zweite Zusammensetzung in Form eines Nagellacks, eines Wangenrouges, eines Lidschattens, eines Make-ups oder in Form eines Produktes zum Schminken der Lippen oder des Körpers vorliegt.

41. Kit zum Schminken nach einem der Ansprüche 23 bis 40, **dadurch gekennzeichnet**, dass er Mittel zum Auftragen der ersten und der zweiten Zusammensetzung auf die Haut und/oder die Hautanhangsgebilde aufweist.

42. Kit zum Schminken nach Anspruch 41, **dadurch gekennzeichnet**, dass die Mittel unter Pinseln, Bürsten, Federn und Schwämmen ausgewählt sind.

43. Kit zum Schminken nach einem der Ansprüche 23 bis 42, **dadurch gekennzeichnet**, dass die erste und die zweite Zusammensetzung in unterschiedlichen Kompartimenten oder Behältern konfektioniert sind.

44. Kit zum Schminken nach einem der Ansprüche 23 bis 43, **dadurch gekennzeichnet**, dass die erste filmbildende Zusammensetzung ein in einem wässerigen Medium dispergiertes Styrol/Acrylat-Copolymer oder in einem organischen Lösemittelmedium solubilisierte Nitrocellulose enthält.

45. Kit zum Schminken nach einem der Ansprüche 23 bis 44, **dadurch gekennzeichnet**, dass die zweite filmbildende Zusammensetzung ein Styrol/Acrylat-Copolymer enthält, das in einem wässerigen Medium dispergiert ist.

46. Geschminkter Träger, der eine Schminke aufweist, die gemäß dem Verfahren nach einem der Ansprüche 1 bis 21 erhalten werden kann.

47. Träger nach Anspruch 46, **dadurch gekennzeichnet**, dass er in Form von falschen Nägeln, falschen Wimpern, Toupets, Perücken, Plättchen oder Haftpflästerchen für die Haut oder die Lippen vorliegt.

48. Verwendung einer ersten filmbildenden Zusammensetzung, die ein kosmetisch akzeptables Medium und mindestens ein erstes filmbildendes Polymer enthält, wobei die erste Zusammensetzung zur Bildung eines ersten Films mit einer Härte d1 im Bereich von 20 bis 85 Sekunden befähigt ist,
und einer zweiten filmbildenden Zusammensetzung, die ein kosmetisch akzeptables Medium und mindestens ein zweites filmbildendes Polymer enthält, wobei die zweite Zusammensetzung zur Bildung eines zweiten Films mit einer Härte d2 befähigt ist, die so ist, dass d2 - d1 größer oder gleich 10 Sekunden ist,
wobei die Härte der Filme gemäß der Norm NF-T 30-016 mit einem Pendelschlagwerk nach Persoz bestimmt wird,
um eine Schminke mit rissiger Oberfläche und guter Beständigkeit zu erhalten.

49. Verwendung nach Anspruch 48, **dadurch gekennzeichnet**, dass die erste filmbildende Zusammensetzung ein in einem wässerigen Medium dispergiertes Styrol/Acrylat-Copolymer oder in einem organischen Lösemittelmedium solubilisierte Nitrocellulose enthält,
und dass die zweite filmbildende Zusammensetzung ein Styrol/Acrylat-Copolymer enthält, das in einem wässerigen Medium dispergiert ist.

50. Verwendung einer filmbildenden Zusammensetzung, die ein kosmetisch akzeptables Medium und mindestens ein erstes filmbildendes Polymer enthält, wobei die Zusammensetzung zur Bildung eines ersten Films mit einer gemäß der Norm NF-T 30-016 mit einem Pendelschlagwerk nach Persoz bestimmten Härte d1 im Bereich von 20 bis 85 Sekunden befähigt ist, um eine Schminke mit rissiger Oberfläche und guter Beständigkeit zu erhalten.

51. Verwendung nach Anspruch 50, **dadurch gekennzeichnet**, dass die erste filmbildende Zusammensetzung ein in einern wässerigen Medium dispergiertes Styrol/Acrylat-Copolymer oder in einem organischen Lösemittelmedium solubilisierte Nitrocellulose enthält.

52. Verwendung einer filmbildenden Zusammensetzung in einem Schminkprodukt, die ein kosmetisch akzeptables Medium und mindestens ein filmbildendes Polymer enthält und die dazu bestimmt ist, auf einen ersten Film mit einer Härte d1 im Bereich von 20 bis 85 Sekunden aufgetragen zu werden,
wobei die Zusammensetzung zur Bildung eines zweiten Films mit einer Härte d2 befähigt ist, die so ist, dass d2 - d1 größer oder gleich 10 Sekunden ist, wobei die Härte der Filme gemäß der Norm NF-T 30-016 mit einem Pendelschlagwerk nach Persoz bestimmt wird,
um eine Schminke mit rissiger Oberfläche und guter Beständigkeit zu erhalten.

53. Verwendung nach Anspruch 52, **dadurch gekennzeichnet**, dass die filmbildende Zusammensetzung ein Styrol/Acrylat-Copolymer enthält, das in einem wässerigen Medium dispergiert ist.
